# DEMANDE DE BREVET EUROPEEN

(11) **EP 1 022 013 A1**
(43) Date de publication de la demande: **26.07.2000**
(21) Numéro de dépôt: 00400178.0
(22) Date de dépôt: 24.01.2000
(51) Int. Cl.: A61K 7/00

(54) **Composition cosmétique anhydre essentiellement exempte de liant gras et procédé de préparation d'une telle composition**

(30) Priorité: 25.01.1999 FR 9900764
(71) Demandeur: MERCK S.A., 94000 Nogent-sur-Marne (FR)
(72) Inventeur: Grandmontagne, Bernard, 29880 Plouguerneau (FR); Vouzellaud, Laure, 92120 Montrouge (FR); Marchio, Francois, 75020 Paris (FR)
(74) Mandataire: Ahner, Francis

(57) **Abrégé**

La présente invention concerne une composition cosmétique anhydre essentiellement exempte de liant gras, caractérisée en ce qu'elle comprend une forme compactée de microparticules de pigments minéraux enrobées d'une couche de chitine ou dérivés de chitine et éventuellement une ou plusieurs charges usuelles en cosmétique.

Application à la réalisation de rouge à lèvres, stick à lèvres, stick anti-cernes.

## Description

La présente invention concerne une composition cosmétique anhydre essentiellement exempte de liant gras ainsi qu'un procédé de préparation d'une telle composition.

Les compositions cosmétiques selon l'invention sont essentiellement celles concernant le maquillage du visage, c'est-à-dire les fards à paupières, fonds de teint, fards à joues, « blush », rouges à lèvres.

La demande de brevet WO-A-98/22540 décrit des microparticules de pigments minéraux enrobés d'une couche de chitine ou de dérivés de chitine et un procédé de préparation de ces microparticules.

Cette demande de brevet décrit également les propriétés remarquables des formes agrégées ou compactées de ces microparticules quant à leur capacité de charge élevée et donc leur aptitude à « véhiculer » des substances, notamment toxiques dans le domaine phytosanitaire.

Cette demande de brevet fait également état d'applications filmogènes diverses telles que l'enrobage des semences, l'enrobage de galets, de comprimés, la formulation des encres et des peintures.

Le matériau décrit sous la forme de « compact » est issu de la concrétion de pigments enrobés de chitine liés entre eux par liaisons hydrogène.

La cohésion des particules minérales, en particulier les pigments lamellaires de type mica-titane est assurée par la qualité du film chitineux de surface et les conditions d'évaporation de l'eau.

Cette structure lamellaire confère au matériau un comportement spongieux non élastique, à maille aérée dont les interstices forment des cavités d'un volume total voisin de 90 % du matériau et qui sont aptes à être comblées par toute autre molécule ou formation de molécules incluses lors du processus de formation.

Selon cette demande de brevet, d'autres utilisations peuvent également être envisagées telles que le compactage des colorants, des pigments ou encore des parfums. Notamment le toucher agréable des microparticules selon l'invention permet leur usage dans la formulation à sec des industries cosmétiques (fond de teint).

Les compositions cosmétiques anhydres qui se présentent sous forme de poudre compactée en plus d'un support acceptable en cosmétique nécessitent la présence d'un liant gras afin d'assurer la cohésion de la poudre.

Ces liants sont bien connus et on en trouvera une liste dans la demande de brevet EP-A-815 826, page 8, ligne 40 à page 9, ligne 5.

Les supports usuels sous forme de poudre sont notamment le talc.

Par ailleurs des compositions de bâtons de rouge à lèvres ou de lipsticks à base de liants gras sont notamment décrites dans la demande de brevet WO-A-95/11000.

Il est donc un fait établi que dans le cas des formulations cosmétiques sous forme de poudre, il est essentiel de maintenir ou d'améliorer la cohésion de la poudre compactée par l'incorporation d'un liant gras.

Il est néanmoins souhaitable de s'affranchir de ces liants gras afin d'améliorer le confort des utilisatrices, notamment en ce qui concerne le démaquillage. En effet, les liants gras présentent une adhésion relativement forte à la peau ce qui conduit à allonger le démaquillage. Par ailleurs, ces liants peuvent occasionner d'éventuelles taches aux vêtements résultant d'une mauvaise utilisation des bâtons de rouge à lèvres.

De façon surprenante, on a trouvé que les formes compactées de microparticules telles que mentionnées ci-dessus permettaient de s'affranchir de la présence de liants gras tout en conduisant à des compositions cosmétiques anhydres solides non élastiques offrant un compromis parfait entre résistance physique et capacité à libérer un tracé homogène et filmogène sur la peau.

De plus, ces compositions cosmétiques peuvent être essuyées par un simple lavage à l'eau.

Ainsi, les fonds de teint, fards à paupière et blush peuvent se présenter sous forme de craies non grasses, résistantes à la cassure et capables de s'appliquer directement sur les parties du corps ou du visage destinées au maquillage.

L'application directe de ces craies dispense donc de l'emploi d'applicateurs et ustensiles de prélèvement divers.

Selon l'invention, les compositions cosmétiques anhydres essentiellement exemptes de liant gras sont caractérisées en ce qu'elles comprennent une forme compactée de microparticules de pigments minéraux enrobés d'une couche de chitine ou dérivés de chitine et, éventuellement, une ou plusieurs charges usuelles en cosmétique.

Par « chitine », au sens de la présente demande, on entend « chitosan réacétylé », aussi ce terme de « chitine » ne doit pas être compris comme définissant ni le chitosan en tant que tel, ni la chitine native (naturelle).

Les microparticules de pigments minéraux enrobés sont décrites dans la demande de brevet WO-A-98/22540 du même déposant.

Leur procédé de préparation est également décrit dans cette demande de brevet.

De préférence, lesdites microparticules de pigments minéraux non enrobées présentent une granulométrie moyenne comprise entre 1 et 200 µm, de préférence une granulométrie moyenne supérieure à 5 µm.

Les pigments minéraux sont des substances naturelles ou de synthèse constituées de fines particules dont la fonction principale consiste à donner une coloration.

Parmi ces pigments minéraux, on cite notamment les oxydes métalliques et les pigments nacrés.

Parmi les oxydes métalliques, on cite notamment les oxydes de zirconium, de cérium, de zinc ou de chrome (Cl 77 288), le dioxyde de titane (Cl 77 891), les oxydes de fer noir, jaune, rouge et brun (Cl 77 499, Cl 77492, Cl 77491), le violet de manganèse (Cl 77 742), le bleu outremer (Cl 77 007), le bleu ferrique (Cl 77 510), l'hydrate de chrome (CL 77 289), la poudre d'argent ou la poudre d'aluminium, les silicates, les silicates traités, le sulfate de baryum, les talcs, les bromo-nitrates.

Parmi les pigments nacrés, on cite l'oxychlorure de bismuth, le mica recouvert d'oxyde de titane ou d'oxyde de fer ou le mica-titane coloré.

De nombreuses charges usuelles en cosmétique peuvent être incorporées dans la structure matricielle des formes compactées sans altérer les propriétés physiques desdites compositions.

Parmi ces charges, on cite notamment les substances actives pour l'entretien de la peau. Parmi ces substances, on cite les vitamines telles que la vitamine A et E, les écrans solaires et les matières pharmaceutiquement actives. Parmi ces matières actives, on cite notamment l'oxyde de zinc, l'huile de chamomile, l'extrait de ginko biloba et d'autres matières actives équivalentes. Ces charges sont usuellement présentes en quantité inférieure à 2 % en poids et généralement entre 0,1 % à 1 % en poids.

On cite également les parfums.

L'une des caractéristiques particulièrement remarquable et inattendue réside dans le fait que ces formes compactées de microparticules offrent un compromis parfait entre résistance physique et capacité à libérer un tracé homogène et filmogène sur la peau. Ainsi, les fonds de teint, fards à paupières et blush peuvent être présentés sous forme de craies non grasses, résistantes à la cassure et capables de s'appliquer directement sur les parties du corps ou du visage destinées au maquillage. L'application directe de ces craies dispense de l'emploi d'applicateurs et ustensiles de prélèvement divers.

C'est la raison pour laquelle l'invention concerne une composition cosmétique anhydre telle que définie précédemment sous la forme de bâtonnet.

L'invention trouve une application particulièrement remarquable dans le cas des rouges à lèvres, des sticks à lèvres ou des sticks anti-cernes ou analogues.

Dans ce cas, la possibilité de produire des crayons (en craies) à lèvres sans composé gras présente de multiples avantages.

En d'autres termes, les particularités d'application des formes compactées selon l'invention sont caractérisées par :
- un tracé homogène et filmogène lié à une libération adéquate et orientée des particules en surface cutanée.
- un toucher « cosmétique », soyeux, accompagné d'un glissant optimal,
- une substantivé des pigments enrobés malgré l'absence de composés lipophiles,
- une possibilité de démaquillage à l'eau.

L'invention concerne également un procédé de préparation d'une composition cosmétique selon l'invention, caractérisé en ce que l'on met en contact les microparticules de pigments minéraux enrobés d'une couche de chitine ou dérivés de chitine, éventuellement en présence d'une ou plusieurs charges, avec une quantité d'eau appropriée, en ce qu'on élimine l'eau par compactage dans un moule approprié et en ce qu'éventuellement on soumet la forme compactée à un séchage.

Le procédé de préparation consiste donc en la réhydratation de pigments enrobés de chitine précédemment décrits, jusqu'à obtention d'une pâte de consistance telle qu'elle puisse être coulée, agitée et formulée avec diverses charges.

Les teneurs en eau nécessaires à la réalisation de pâtes sont variables et adaptables à chaque formule. Dans tous les cas, il est nécessaire que la quantité d'eau soit inférieure à la quantité nécessaire à la remise en suspension liquide des microparticules.

La seconde étape est le moulage de cette pâte dans un contenant tel qu'il assure la forme finale requise. Ce peut être des formettes en bâtonnet, stick ou toute autre forme obtenue par moulage et démoulage. L'étape finale est le séchage. La pâte perd ainsi son eau et on obtient une cohésion suffisante de la forme moulée.

Le séchage peut être effectué par un essorage de la pâte moulée, une centrifugation ou une compression de telle sorte que l'eau de percolation soit expulsée plus rapidement. Toutes ces techniques sont connues de l'homme du métier. L'essorage est de préférence suivi d'un séchage par chauffage complémentaire.

L'invention est maintenant illustrée par l'exemple suivant.

100 g de pigments enrobés de chitine sont mélangés à 250 ml d'eau pour obtenir une pâte fluide. Le mélange est versé dans un cylindre fermé à une extrémité par une surface filtrante escamotable et équipé à l'autre extrémité d'un piston. Une pression est appliquée au piston ce qui provoque l'expulsion de l'eau au travers du filtre. Le fond filtrant escamoté, un cylindre peut être aisément extrait du système.

La pâte réalisée est versée dans une centrifugeuse, le culot de centrifugation est ensuite retiré. Un séchage élimine l'humidité résiduelle.

Un bâtonnet aux extrémités enrobées de coton (coton tige) ou de toutes autres matières appropriées est plongé dans la pâte. Le bâtonnet est soutiré et le pigment s'auto-adhère aux extrémités en donnant un système de maintien et d'application.

L'ensemble de ces techniques peut être appliqué aux pigments enrobé chitine ou à des formulations de ces pigments.

## Revendications

1. Composition cosmétique anhydre essentiellement exempte de liant gras, caractérisée en ce qu'elle comprend une forme compactée de microparticules de pigments minéraux enrobées d'une couche de chitine ou dérivés de chitine et éventuellement une ou plusieurs charges usuelles en cosmétique.

2. Composition cosmétique selon la revendication 1, caractérisée en ce que lesdites microparticules de pigments minéraux non enrobées présentent une granulométrie moyenne comprise entre 1 et 200 µm.

3. Composition cosmétique selon la revendication 2, caractérisée en ce que lesdites microparticules de pigments minéraux non enrobées présentent une granulométrie moyenne supérieure à 5 µm.

4. Composition cosmétique selon l'une des revendications précédentes, caractérisée en ce que lesdites microparticules de pigments minéraux non enrobées sont choisies dans le groupe constitué par les oxydes métalliques et les pigments nacrés.

5. Composition cosmétique selon la revendication 4, caractérisée en ce que les oxydes métalliques sont choisis dans le groupe constitué par les oxydes de zirconium, de cérium, de zinc ou de chrome (Cl 77 288), le dioxyde de titane (Cl 77 891), les oxydes de fer noir, jaune, rouge et brun (CI 77 499, CI 77492, CI 77491), le violet de manganèse (CI 77 742), le bleu outremer (Cl 77 007), le bleu ferrique (Cl 77 510), l'hydrate de chrome (CL 77 289), la poudre d'argent ou la poudre d'aluminium, les silicates, les silicates traités, le sulfate de baryum, les talcs, les bromo-nitrates.

6. Composition cosmétique selon la revendication 4, caractérisée en ce que les pigments nacrés sont choisis dans le groupe constitué par l'oxychlorure de bismuth, le mica recouvert d'oxyde de titane, d'oxyde de fer, ou le mica titane coloré.

7. Composition cosmétique selon l'une des revendications précédentes, caractérisée en ce que les charges sont choisies parmi les parfums, les substances actives pour le soin de la peau, notamment les vitamines.

8. Composition cosmétique selon l'une des revendications précédentes, caractérisée en ce qu'elle se présente sous la forme de bâtonnets.

9. Composition cosmétique selon la revendication 8, caractérisée en ce qu'elle se présente sous la forme de rouge à lèvres, de stick à lèvres ou de stick anti-cernes.

10. Procédé de préparation d'une composition cosmétique selon l'une des revendications précédentes, caractérisée en ce qu'on met en contact les microparticules de pigments minéraux enrobés d'une couche de chitine ou dérivés de chitine, éventuellement en présence d'une ou plusieurs charges, avec une quantité d'eau appropriée, on élimine l'eau par compactage dans un moule approprié et éventuellement on procède à un séchage.
